# EUROPÄISCHE PATENTANMELDUNG

(11) **EP 1 496 047 A1**
(43) Veröffentlichungstag der Anmeldung: **12.01.2005**
(21) Anmeldenummer: 04021980.0
(22) Anmeldetag: 02.02.2001
(51) Int. Cl.: C07C 231/04, C07C 237/16, C07C 237/10, C07C 231/12

(54) **Verfahren zur Herstellung von Enaminen acetoacetylierter aromatischer Amine**

(30) Priorität: 04.02.2000 EP 00102418; 12.05.2000 US 203922 P
(62) Teilanmeldung aus: 01913783.5
(71) Anmelder: Lonza AG, 4052 Basel (CH)
(72) Erfinder: Glufke, Uta, 4056 Basel (CH); Hanselmann, Paul, 3902 Brig-Glis (CH)

(57) **Zusammenfassung**

Die Erfindung betrifft ein Verfahren zur Herstellung von Verbindungen der allgemeinen Formel 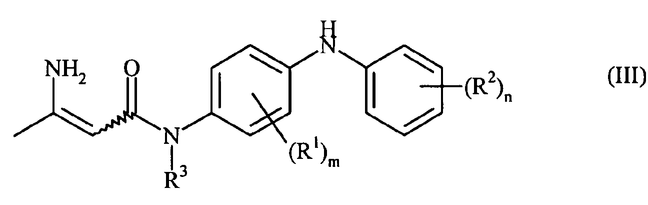 worin
- R¹ und R²: bei jedem Auftreten unabhängig voneinander jeweils Hydroxy, C₁₋₆-Alkyl, C₁₋₆-Alkoxy, Halogen, Phenyl oder Phenoxy;
- R³: C₁₋₆-Alkyl;
- m: eine ganze Zahl von 0 bis 4; und
- n: eine ganze Zahl von 0 bis 5 bedeuten,
wobei Diketen mit einem N-Phenyl-p-phenylendiamin der allgemeinen Formel 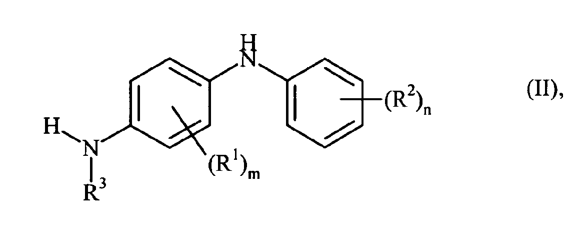 umgesetzt und das Produkt mit Ammoniak zur Reraktion gebracht wird. Die Verbindungen als solche und ihre Hydrierungsprodukte sind ebenfalls Gegenstand der Erfindung.

## Beschreibung

Die vorliegende Erfindung betrifft ein Verfahren zur Herstellung von Enaminen acetoacetylierter aromatischer Amine, insbesondere von Enaminen acetoacetylierter *N*-Phenyl-*p*-phenylendiamine, sowie neue Enamine acetoacetylierter *N*-Phenyl-*p*-phenylendiamine und deren Hydrierungsprodukte.

US Patent Nr. 2 115 413 beschreibt die Umsetzung von Acetessigsäureethylester mit *N*-Phenyl-*p*-phenylendiamin zur Herstellung von 3-Oxo-*N*-[4-(phenylamino)-phenyl]butyramid.

Die Umsetzung von primären aromatischen Aminen mit Diketen zur Herstellung von Acetoacetaniliden beschreiben C. E. Kaslow et al. in *J. Am. Chem*. *Soc*. **1946,** *68*, 644-647.

Die Umsetzung von sekundären aromatischen Aminen mit Diketen zur Herstellung von N-Alkylacetoacetaniliden beschreiben C. E. Kaslow et al. in *J*. *Am*. *Chem*. *Soc*. **1945,** *67*, 1969-1970. Die Umsetzung wird auch von N. Etkin et al. in *J*. *Org. Chem*. **1990,** *55,* 1093-1096 beschrieben. Die hierbei eingesetzten sekundären aromatischen Amine sind alkyl- oder alkoxysubstituierte Aniline. Die Umsetzung von *N*-C₁₋₆-Alkyl-3-oxo-*N*-[4-(phenylamino)phenyl]butyramiden wird nicht beschrieben.

Aufgabe der vorliegenden Erfindung war es, ein Verfahren zur Herstellung von Enaminen acetoacetylierter *N*-Phenyl-*p*-phenylendiamine bereitzustellen.

Diese Aufgabe wird durch das Verfahren gemäss Patentanspruch 1 gelöst.

Das Verfahren betrifft die Herstellung von Verbindungen der allgemeinen Formel 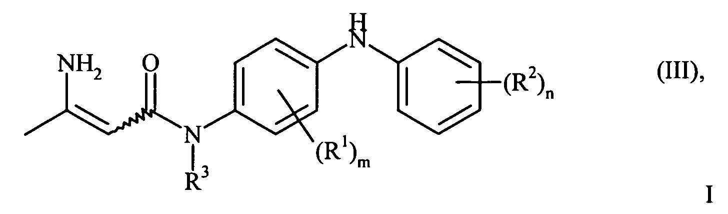 worin
- R¹ und R²: bei jedem Auftreten unabhängig voneinander jeweils Hydroxy, C₁₋₆-Alkyl, C₁₋₆-Alkoxy, Halogen, Phenyl oder Phenoxy;
- R³: C₁₋₆-Alkyl;
- m: eine ganze Zahl von 0 bis 4; und
- n: eine ganze Zahl von 0 bis 5 bedeuten.

Das Verfahren ist dadurch gekennzeichnet, dass zunächst Diketen mit einem *N*-Phenyl-*p*-phenylendiamin der allgemeinen Formel 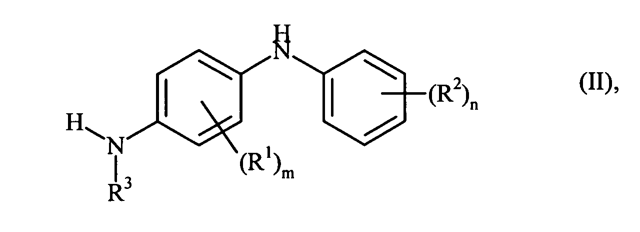 worin R¹, R², R³, m und n die oben angegebene Bedeutung haben, in Gegenwart von 3-40%iger Essigsäure bei Temperaturen von 20 bis 100 °C, vorzugsweise bei 60 bis 70 °C zu einer Verbindung der allgemeinen Formel 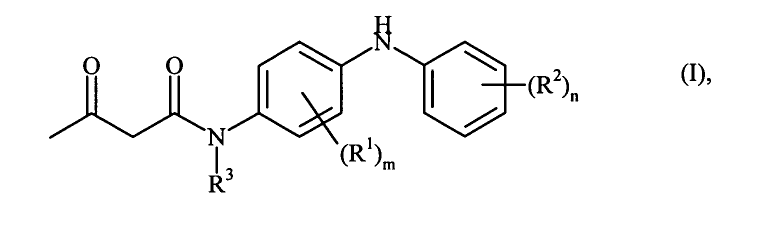 umgesetzt wird.

Diese Verbindungen können in mindestens zwei tautomeren Formen (Keto/Enol-Tautomerie) vorliegen, wobei hier zur Vereinfachung jeweils nur die Ketoform abgebildet ist.

Unter C₁₋₆-Alkyl sind hier und im folgenden alle linearen oder verzweigten Alkylgruppen mit 1-6 Kohlenstoffatomen zu verstehen, wie z. B. Methyl, Ethyl, Propyl, Isopropyl, Butyl, Isobutyl, *sec*-Butyl, *tert*-Butyl, Pentyl, Isopentyl, *tert*-Pentyl, Neopentyl, Hexyl oder Isohexyl.

Unter C₁₋₆-Alkoxy sind Gruppen, die aus C₁₋₆-Alkyl und Sauerstoff zusammengesetzt sind, zu verstehen, wie z. B. Methoxy, Ethoxy, Propoxy, Isopropoxy, Butoxy, *sec*-Butoxy oder *tert*-Butoxy.

Die Variablen m und n haben vorzugsweise den Wert 0 (Null). Der Rest R³ ist C₁₋₆-Alkyl, besonders bevorzugt Isopropyl.

Überraschenderweise erfolgt die Reaktion des *N*-Phenyl-*p*-phenylendiamins (II) mit Diketen nur am Stickstoffatom, welches den Rest R³ trägt.

Die Verbindungen der Formel II, worin R³ Wasserstoff bedeutet, können nach bekannten Methoden, z. B. durch Umsetzung von Anilin oder substituierten Anilinen mit Nitrobenzol oder Azobenzol und anschliessende Hydrierung, hergestellt werden. Die Verbindungen der Formel II, worin R³ C₁₋₆-Alkyl bedeutet, können nach bekannten Methoden, z. B. durch reduktive Alkylierung von *N*-Phenyl-*p*-phenylendiaminen der Formel II (R³ = H) mit einem aliphatischen Keton oder Aldehyd hergestellt werden.

Die Verbindungen der Formel I werden durch Umsetzung mit Ammoniak in die entsprechenden Enamine der allgemeinen Formel 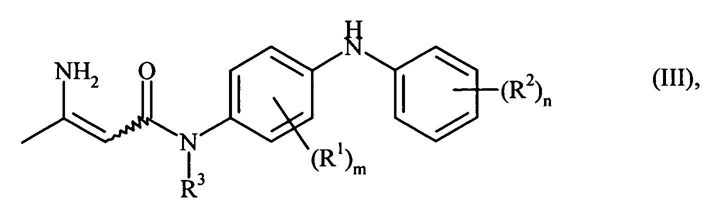 worin R¹, R², R³, m und n die oben angegebene Bedeutung haben, umgewandelt. Diese Enamine können in zwei geometrischen Isomeren (*E* und *Z*-Konfiguration an der Enamin-Doppelbindung) vorliegen, wobei hier sowohl die beiden Isomeren als auch deren Gemische umfasst sind. Vorzugsweise wird die *Z*-Konfiguration gebildet.

Die Umsetzung mit Ammoniak erfolgt vorteilhaft bei Temperaturen von 10 bis 150 °C, vorzugsweise etwa 70 °C, und Drücken von 1 bis 100 bar, vorzugsweise 10 bis 30 bar. Die Umsetzung erfolgt vorteilhaft in einem geeigneten Lösungsmittel in Gegenwart von katalytischen Mengen an konzentrierter oder wässriger Essigsäure. Geeignete Lösungsmittel sind beispielsweise Ester, aromatische und aliphatische Kohlenwasserstoffe, chlorierte aliphatische Kohlenwasserstoffe, Ether und Polyether, Alkohole sowie Wasser. Bevorzugt wird Ethylacetat als Lösungsmittel eingesetzt. Vorteilhaft werden 0,02-2,0 mol konzentrierte Essigsäure auf 1 mol der Verbindung der Formel I eingesetzt.

Die Verbindungen der Formel III sind neu und ebenfalls Gegenstand der Erfindung.

Durch katalytische Hydrierung der Verbindungen der Formel III entstehen Verbindungen der allgemeinen Formel 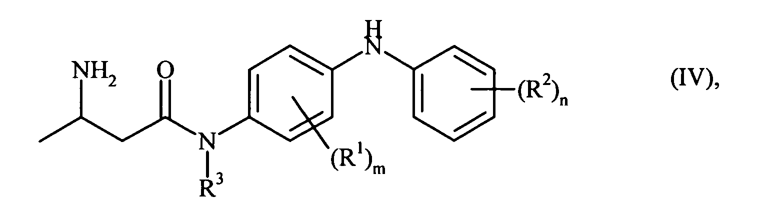 worin R¹, R², R³, m und n die oben angegebene Bedeutung haben.

Die katalytische Hydrierung erfolgt vorteilhaft bei Temperaturen von 100 bis 200 °C, vorzugsweise etwa 110 °C, und Drücken von 20 bis 150 bar, vorzugsweise etwa 100 bar. Ein geeigneter Hydrierkatalysator ist beispielsweise Raney-Nickel. Die Hydrierung erfolgt vorteilhaft in einem geeigneten Lösungsmittel in Gegenwart einer Base, vorzugsweise in Gegenwart von Ammoniak. Geeignete Lösungsmittel sind beispielsweise aliphatische Alkohole oder Mischungen von Wasser und aliphatischen Alkoholen, bevorzugt wird Methanol eingesetzt.

Die Verbindungen der Formel IV sind neu und ebenfalls Gegenstand der Erfindung.

Die folgenden Beispiele verdeutlichen die Durchführung des erfindungsgemässen Verfahrens und die Herstellung der erfindungsgemässen Verbindungen, ohne dass darin eine Einschränkung zu sehen ist.

### Beispiel 1

### N-Isopropyl-3-oxo-N-[4-(phenylamino)phenyl]butyramid (I, m = n = 0, R³ = Isopropyl)

Diketen (261,10 g, 3,105 mol) wurde zu einer Lösung vom *N*-(Isopropylamino)-*N*-phenyl-*p*-phenylendiamin (388,00 g, 1,714 mol) in 40%iger Essigsäure (1200 ml) während 2 h bei 60 °C zugetropft. Nach der Zugabe des Diketens wurde das Reaktionsgemisch 2,5 h bei 65 °C gerührt und über Nacht auf 5 °C gekühlt. Der entstandene Niederschlag wurde abgenutscht, mit Wasser (2×150 ml) gewaschen und im Vakuum (20 mbar) bei 40 °C getrocknet. Man erhielt 400,60 g (75% Ausbeute) *N*-Isopropyl-3-oxo-*N*-[4-(phenylamino)phenyl]butyramid als dunkelgrauen Feststoff.
¹H NMR (400 MHz, CDCl₃) δ = 14,48 (s, 0,1 H, O*H*_{Enol}); 7,35-7,25 (m, 2 H, Ar-*H*); 7,20-7,10 (m, 2 H, Ar-*H*); 7,10-6,95 (m, 3 H, Ar-*H*); 6,95-6,90 (m, 2 H, Ar-*H*); 6,01 (s, 1 H, N*H*); 5,05-4,92 (m, 1 H, C*H*(CH₃)₂); 4,45 (s, 0,1 H, CO-C*H*=COH); 3,21 (s, 1,9 H, CO-C*H*₂-CO); 2,10 (s, 2,7 H, C*H*_{3 Keto}); 1,89 (s, 0,3 H, C*H*_{3 Enol}); 1,09 (d, *J*= 8,2 Hz, 6 H, CH(C*H*₃)₂).

### Beispiel 2

### (Z)-3-Aminobut-2-ensäure-N-isopropyl-N-[4-(phenylamino)phenyl]amid

### (III, m = n = 0, R³ = Isopropyl)

In einem Autoklaven wurden *N*-Isopropyl-3-oxo-*N*-[4-(phenylamino)phenyl]butyramid (30,0 g, 0,0967 mol) und konz. Essigsäure (1,4 g, 0,0233 mol) in Ethylacetat (140 g) vorgelegt. Innerhalb von 10 min wurde Ammoniak (16,0 g, 0,9395 mol) eingeleitet und die Temperatur des Reaktionsgemisches während dieser Zeit langsam auf 35 °C erhöht. Nach dem Einleiten betrug der Druck 14 bar. Das Reaktionsgemisch wurde dann innerhalb von 30 min auf 70 °C erhitzt, wobei der Druck auf 17 bar stieg. Nach weiteren 2,5 h unter diesen Bedingungen wurde das Reaktionsgemisch auf 25 °C abgekühlt und das überschüssige Ammoniak abgelassen. Das Reaktionsgemisch wurde weiter auf 5 °C gekühlt, um das Produkt zu kristallisieren. Die Suspension wurde filtriert und der erhaltene hellgraue Feststoff mit 0 °C kaltem Ethylacetat (20 ml) gewaschen und 2 Tage bei 35 °C im Vakuum (50 mbar) getrocknet. Man erhält (2)-3-Aminobut-2-ensäure-*N*-isopropyl-*N*-[4-(phenylamino)phenyl]amid (27,2 g, 91,0%).
¹H NMR (400 MHz, CDCl₃) δ = 7,36-7,25 (m, 2 H, Ar-*H*); 7,18-7,11 (m, 2 H, Ar-*H*); 7,09-7,02 (m, 2 H, Ar-*H*); 6,99-6,96 (m, 3 H, Ar-*H*); 5,84 (s, 1 H, N*H*); 5,05-4,99 (m, 1 H, C*H*(CH₃)₂); 4,04 (s, 1 H, CO-C*H*=C); 1,71 (s, 3 H, C*H*₃); 1,03 (d, *J* = 8 Hz, 6 H, CH(C*H*₃)₂).

### Beispiel 3

### 3-Amino-N-isopropyl-N-[4-(phenylamino)phenyl]butyramid

### (IV, m = n = 0, R³ = Isopropyl)

In einem Autoklaven wurden (*Z*)-3-Aminobut-2-ensäure-*N*-isopropyl-*N*-[4-(phenylamino)phenyl]amid (13,9 g, 0,0449 mol) und Raney-Nickel (K0840 Ni B113W) (2,0 g) in Methanol (140 g) vorgelegt. Innerhalb von 5 min wurde Ammoniak (5,5 g, 0,3230 mol) eingeleitet. Die Temperatur des Reaktionsgemisches betrug 25 °C. Der Druck stieg auf 5 bar. Nach dem Einleiten von Ammoniak wurde das Reaktionsgemisch auf 80 °C erwärmt (kein weiterer Druckanstieg). Bei 80 °C wurde Wasserstoff aufgepresst, bis der Druck 100 bar betrug. Das Reaktionsgemisch wurde auf 110 °C erwärmt und während 4,5 h bei dieser Temperatur gerührt. Hierbei wurde ein Druck von 100 bar durch kontinuierliches Aufpressen von Wasserstoff aufrechterhalten. Dann wurde das Reaktionsgemisch auf 25 °C abgekühlt und der Druck vorsichtig abgelassen. Das Reaktionsgemisch wurde filtriert und das Filtrat zur Hälfte eingeeingt. Das Konzentrat wurde mit 1 g Aktivkohle versetzt, 1 h gerührt, filtriert und zur Trockne eingeengt. Man erhielt 12,8 g Rohprodukt, welches bei 175 °C geschmolzen wurde. Die Schmelze liess man auf einer Aluminiumfolie auskristallisieren. Man erhielt 11,1g (79,3%) 3-Amino-*N*-isopropyl-*N*-[4-(phenylamino)phenyl]butyramid.
¹H NMR (400 MHz, CDCl₃) δ = 7,38-6,90 (m, 9 H, Ar-*H*); 6,03 (s, 1 H, N*H*); 5,05-4,95 (m, 1 H, C*H*(CH₃)₂); 3,42-3,32 (m, 1 H, C*H*-NH₂); 2,10-1,90 (m, 2 H, COC*H*₂); 1,53 (bs, 2 H, NH₂); 1,03 (d, *J =* 8 Hz, 6 H, CH(C*H*₃)₂); 0,98 (d, *J =* 8 Hz, 3 H, C*H*₃).

## Patentansprüche

1. Verfahren zur Herstellung von Verbindungen der allgemeinen Formel 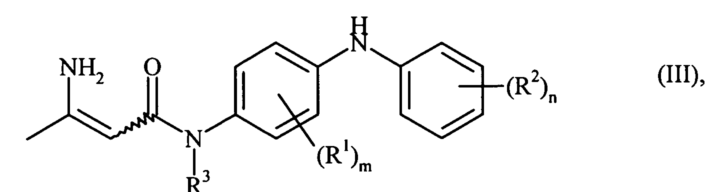 worin
R¹ und R² bei jedem Auftreten unabhängig voneinander jeweils Hydroxy, C₁₋₆-Alkyl, C₁₋₆-Alkoxy, Halogen, Phenyl oder Phenoxy;
R³ C₁₋₆-Alkyl;
m eine ganze Zahl von 0 bis 4; und
n eine ganze Zahl von 0 bis 5 bedeuten,
**dadurch gekennzeichnet, dass** in einer ersten Stufe
Diketen mit einem *N*-Phenyl-*p*-phenylendiamin der allgemeinen Formel 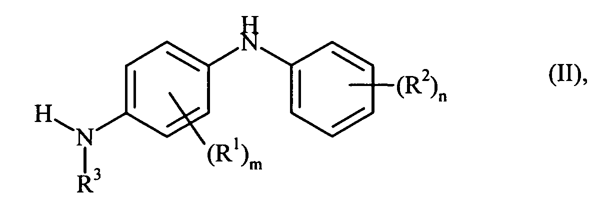 worin R¹, R², R³, m und n die oben angegebene Bedeutung haben, in Gegenwart von 3-40%iger Essigsäure bei Temperaturen von 20 bis 100 °C, vorzugsweise bei 60 bis 70 °C zu einer Verbindung der allgemeinen Formel 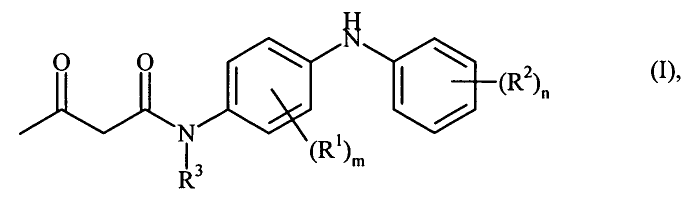 worin R¹, R², R³, m und n die oben angegebene Bedeutung haben, umgesetzt wird, welche in einer zweiten Stufe mit Ammoniak zur Reaktion gebracht wird.

2. Verbindungen der allgemeinen Formel 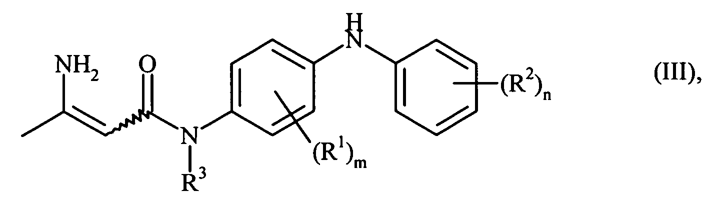 worin R¹, R², R³, m und n die in Anspruch 1 angegebene Bedeutung haben.

3. Verbindungen der allgemeinen Formel 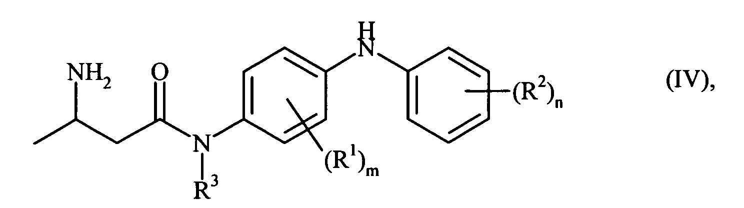 worin R¹, R², R³, m und n die in Anspruch 1 angegebene Bedeutung haben.

4. Verfahren zur Herstellung von Verbindungen der allgemeinen Formel 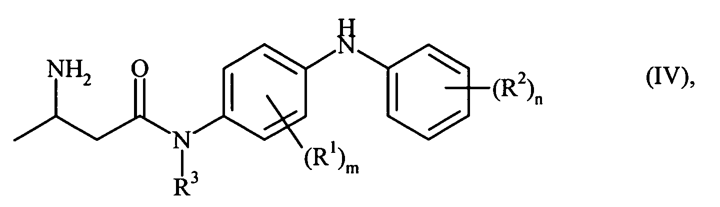 worin R¹, R², R³, m und n die in Anspruch 1 angegebene Bedeutung haben,
**dadurch gekennzeichnet, dass** eine Verbindung der allgemeinen Formel 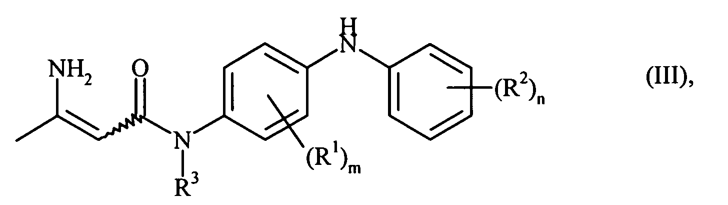 worin R¹, R², R³, m und n die oben genannte Bedeutung haben,
katalytisch hydriert wird.
